# EUROPEAN PATENT APPLICATION

(11) **EP 4 261 844 A1**
(43) Date of publication of application: **18.10.2023**
(21) Application number: 21903189.5
(22) Date of filing: 25.11.2021
(51) Int. Cl.: G16H 50/30, A61B 5/16, A61M 21/00

(54) **INFORMATION PROCESSING DEVICE, INFORMATION PROCESSING METHOD, AND PROGRAM**

(30) Priority: 09.12.2020 JP 2020203933
(71) Applicant: Sony Group Corporation, Tokyo 108-0075 (JP)
(72) Inventor: TERAGUCHI Shinichi, Tokyo 108-0075 (JP); SHIGETA Osamu, Tokyo 108-0075 (JP); KONDO Akane, Tokyo 108-0075 (JP)
(74) Representative: MFG Patentanwälte Meyer-Wildhagen Meggle-Freund Gerhard PartG mbB
(86) International application number: PCT/JP2021/043099
(87) International publication number: WO 2022/124085

(57) **Abstract**

The present disclosure relates to an information processing apparatus, an information processing method, and a program enabling to present more appropriate feedback.

An arousal state determination unit determines which state among hyperarousal, optimal arousal, and hypoarousal an arousal state of a user is in, and a feedback information generation unit generates feedback information for the user on the basis of the determined arousal state. The technology according to the present disclosure can be applied to, for example, a wearable device.

## Description

### TECHNICAL FIELD

The present disclosure relates to an information processing apparatus, an information processing method, and a program, and particularly to an information processing apparatus, an information processing method, and a program enabling to present more appropriate feedback.

### BACKGROUND ART

A technique for determining a physical state and a mental state of a human is known.

Patent Document 1 discloses a technique of calculating an average respiration rate on the basis of a measurement value of respiration, and determining a human arousal state (whether or not drowsiness occurs) on the basis of a change in the calculated average respiration rate.

### CITATION LIST

### PATENT DOCUMENT

Patent Document 1: Japanese Patent Application Laid-Open No. 2018-64759

### SUMMARY OF THE INVENTION

### PROBLEMS TO BE SOLVED BY THE INVENTION

In recent years, there are services for promoting growth of individuals and organizations, such as mental care for the purpose of prevention and treatment of mental disorders and body and mind change. However, these are mainly actions performed by subjective judgment, and an objective mental state index has not been utilized. Therefore, it has been difficult to perform appropriate feedback.

The present disclosure has been made in view of such circumstances, and makes it possible to present more appropriate feedback.

### SOLUTIONS TO PROBLEMS

An information processing apparatus of the present disclosure is an information processing apparatus including an arousal state determination unit that determines which state among hyperarousal, optimal arousal, and hypoarousal an arousal state of a user is in.

An information processing method of the present disclosure an information processing method including, by an information processing apparatus: determining which state among hyperarousal, optimal arousal, and hypoarousal an arousal state of a user is in; and generating feedback information for the user on the basis of the determined arousal state.

A program of the present disclosure is a program causing a computer to execute processing including: determining which state among hyperarousal, optimal arousal, and hypoarousal an arousal state of a user is in; and generating feedback information for the user on the basis of the determined arousal state.

In the present disclosure, it is determined which state among hyperarousal, optimal arousal, and hypoarousal an arousal state of a user is in, and feedback information for the user is generated on the basis of the determined arousal state.

### BRIEF DESCRIPTION OF DRAWINGS

Fig. 1 is a diagram illustrating a paradigm shift of a nervous system.
Fig. 2 is a table for explaining a survival strategy in the Polyvagal theory.
Fig. 3 is a view for explaining an arousal state and a fluctuation of nerve activity.
Fig. 4 is a view illustrating an example of a fluctuation of nerve activity.
Fig. 5 is a view illustrating an example of a fluctuation of nerve activity.
Fig. 6 is a view illustrating an example of widening of a window of tolerance.
Fig. 7 is a diagram illustrating a functional configuration example of an information processing apparatus according to a first embodiment of the present disclosure.
Fig. 8 is a flowchart for explaining a flow of an arousal state determination process.
Fig. 9 is a table illustrating an example of physiological index data.
Fig. 10 is a table illustrating an example of subjective index data.
Fig. 11 is a table for explaining personality factors of the BIG 5.
Fig. 12 is a table illustrating an example of behavioral index data.
Fig. 13 is a flowchart for explaining a flow of a feedback presentation process.
Fig. 14 is a view illustrating an example of a fluctuation of an arousal state.
Fig. 15 is a diagram illustrating a functional configuration example of an information processing apparatus according to a second embodiment of the present disclosure.
Fig. 16 is a flowchart for explaining a flow of a feedback presentation process.
Fig. 17 is a view for explaining an example of estimation of strength of resilience.
Fig. 18 is a view illustrating an example of enhancing strength of resilience.
Fig. 19 is a view for explaining another example of estimation of strength of resilience.
Fig. 20 is a flowchart for explaining a flow of a feedback presentation process.
Fig. 21 is a block diagram illustrating a configuration example of a computer.

### MODE FOR CARRYING OUT THE INVENTION

Hereinafter, embodiments for implementing the present disclosure (hereinafter, referred to as embodiments) will be described. Note that the description will be given in the following order.
1. Conventional problems
2. Polyvagal theory
3. First embodiment of present disclosure (feedback presentation according to arousal state)
4. Second embodiment of present disclosure (feedback presentation according to strength of resilience)
5. Use case
6. Configuration example of computer

### <1. Conventional problems>

While mental care is required socially in a stressful society, in recent years, there are services for promoting growth of individuals and organizations, such as mental care for the purpose of prevention and treatment of mental disorders and body and mind change. However, these are mainly actions performed by subjective judgment, and an objective mental state index has not been utilized. Therefore, it has been difficult to perform appropriate feedback. Furthermore, since a level of improvement of the mental state is not clear, it has been also difficult to maintain long-term motivation.

Specifically, conventionally, a stress index based on a balance theory with two autonomic nerves of a sympathetic nerve and a parasympathetic nerve has been utilized. However, this is insufficient for determining a complicated mental state of a human, and it has been unable to present appropriate feedback.

Therefore, in the technology according to the present disclosure, presentation of appropriate feedback is achieved on the basis of an arousal state of the Polyvagal theory.

### <2.Polyvagal theory>

The Polyvagal theory is a theory relating to an adaptive response of the autonomic nervous system, in which "evolution of mammals" and "social behavior" are linked from the viewpoint of neuroscience, physiology, embryology, and anatomy. The Polyvagal theory is applied not only to the field of neuromedicine but also to therapies for mental disorders and trauma, body work, and training techniques of education sites and the like.

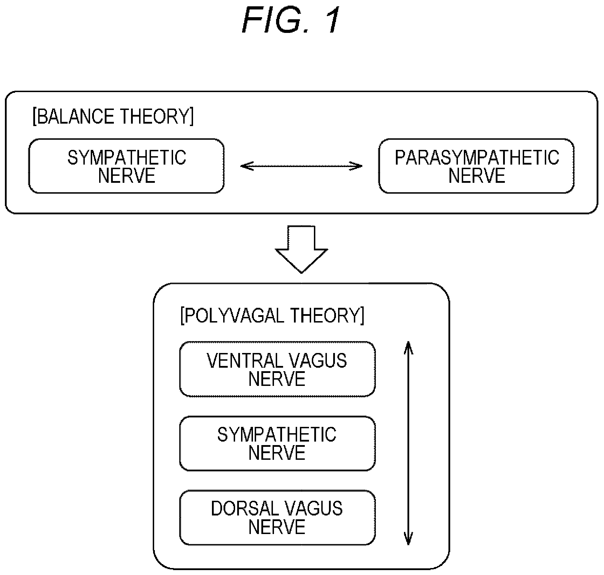

Fig. 1 is a diagram illustrating a paradigm shift of a nervous system.

Conventionally, it has been considered that human self-adjustment and environmental adaptation by the autonomic nerve are performed on the basis of the balance theory with the "sympathetic nerve" and the "parasympathetic nerve". Whereas, in the Polyvagal theory, the "parasympathetic nerve" is divided into a "dorsal vagus nerve" and a "ventral vagus nerve", and it is considered that human self-adjustment and environmental adaptation are performed by three types of autonomic nerves: the "dorsal vagus nerve", the "sympathetic nerve", and the "ventral vagus nerve". According to the Polyvagal theory, these three types of autonomic nerves are considered to have been developed at a stage of evolution of organisms. In the Polyvagal theory, nerve activity is considered to be hierarchical, rather than antagonistic as in balance theory. Note that, the "dorsal vagus nerve" and the "ventral vagus nerve" are formally referred to as a "ventral vagus nerve complex" and a "dorsal vagus nerve complex", respectively, but the terms "dorsal vagus nerve (system)" and "ventral vagus nerve (system)" which are common names are used in the present specification.

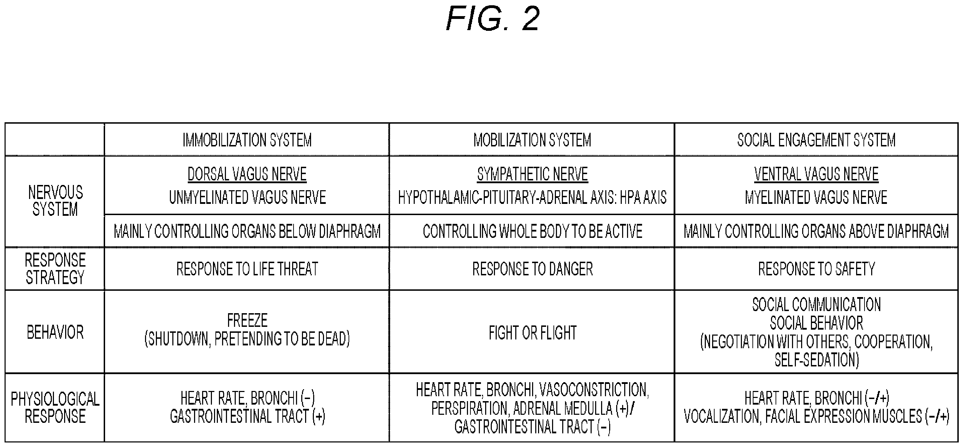

Fig. 2 is a table for explaining a survival strategy in the Polyvagal theory.

Organisms have had appropriate neural survival strategies depending on a stage of evolution. Survival strategies in the Polyvagal theory are defined by three systems corresponding to the "dorsal vagus nerve", the "sympathetic nerve", and the "ventral vagus nerve".

An "immobilization system" functions via the dorsal vagus nerve in the parasympathetic nerve. The dorsal vagus nerve is an unmyelinated vagus nerve, and mainly controls organs below the diaphragm. Development (origin) of the dorsal vagus nervous system is said to be almost all vertebrates after cartilaginous fish. In the "immobilization system", an organism exhibits a "response to life threat" as a response strategy and takes a behavior of "freeze" ("shutdown" or "pretending to be dead"). As physiological responses, for example, a heart rate and bronchi become inactive, and a gastrointestinal tract becomes active.

A "mobilization system" functions by the sympathetic nerve. The sympathetic nerve works by endocrine of the hypothalamic-pituitary-adrenal axis (also referred to as the HPA axis) to control the whole body to be active. Development (origin) of the sympathetic nervous system is said to be animals of bony fish and later. In the "mobilization system", an organism exhibits a "response to danger" as a response strategy, and takes a behavior of "fight or flight". As physiological responses, for example, a heart rate, bronchi, vasoconstriction, perspiration, and adrenal medulla become active, and a gastrointestinal tract becomes inactive.

A "social engagement system" functions via the ventral vagus nerve in the parasympathetic nerve. The ventral vagus nerve is a myelinated vagus nerve, and mainly controls organs above the diaphragm. Development (origin) of the ventral vagus nervous system is said to be animals of mammals and later. In the "social engagement system", an organism exhibits a "response to safety" as a response strategy, and takes a behavior of "social communication" and "social behavior" ("negotiation with others", "cooperation", and "self-sedation") . As physiological responses, for example, a heart rate becomes gentle and stable, vocalization becomes gentle or has intonation, and expression becomes rich.

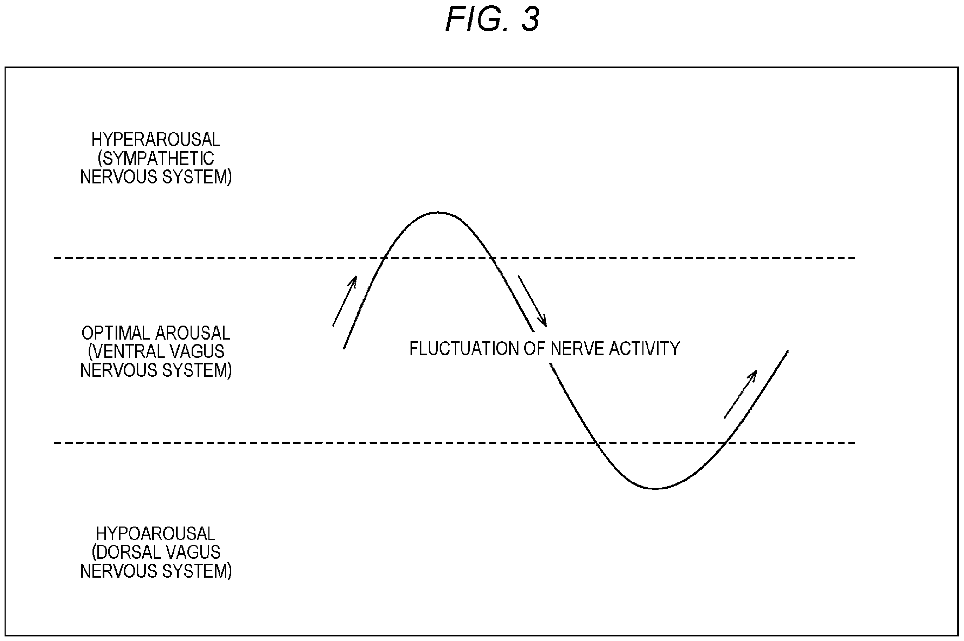

Fig. 3 is a view for explaining an arousal state and a fluctuation of nerve activity.

In the Polyvagal theory, three states of "hypoarousal", "hyperarousal", and "optimal arousal" are defined as arousal states of human by the activity of the three types of autonomic nerves the "dorsal vagus nerve", the "sympathetic nerve", and the "ventral vagus nerve" described above.

In the "hypoarousal", the dorsal vagus nervous system become dominant, and the human survival strategy shifts to the "immobilization system". In this case, the human is in a state of lack of sensation and emotion, lack of vitality, and inability to interact with other people.

In the "hyperarousal", the sympathetic nervous system becomes dominant, and the human survival strategy shifts to the "mobilization system". In this case, the human falls into a state of panic, irritation, impulsiveness, short-sighted thought, and turning aside from unpleasant things.

Whereas, in the "optimal arousal state", the ventral vagus nerve becomes dominant, and the human survival strategy shifts to the "social engagement system". In this case, the human is in a state of adapting to various stimuli, being able to interact with others, and being able to endure.

In Fig. 3, a range of the "optimal arousal state" is a range in which stress can be tolerated, and is also referred to as a window of tolerance or the like. A person having high stress tolerance and a stable balance of the autonomic nerve has a wide window of tolerance, and the nerve activity does not often deviate from the window of tolerance even if the nerve activity fluctuates. On the other hand, a person having low stress tolerance and an unstable balance of the autonomic nerve has a narrow window of tolerance, and the nerve activity fluctuates so as to protrude from the window of tolerance.

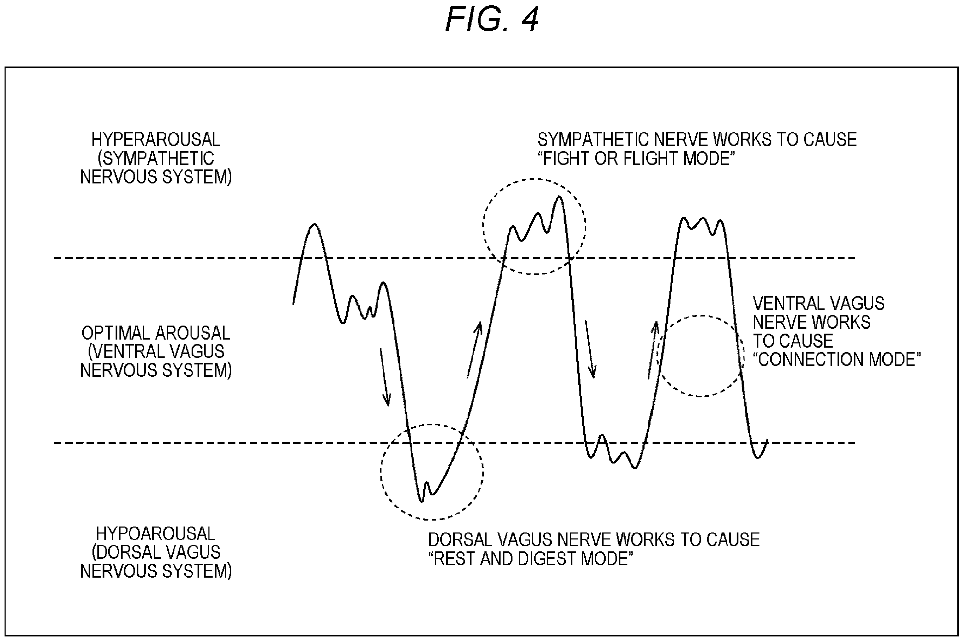

For example, in the example of Fig. 4, the dorsal vagus nerve works to cause a "rest and digest mode", and the sympathetic nerve works to cause a "fight or flight mode", which are repeated. Furthermore, the ventral vagus nerve works between them to cause a "connection mode". In the technology of the present disclosure, as in the example of Fig. 4, in a case where the arousal state repeats hypoarousal and hyperarousal, improvement feedback is presented such that the fluctuation of the nerve activity falls within the optimal arousal state (the window of tolerance).

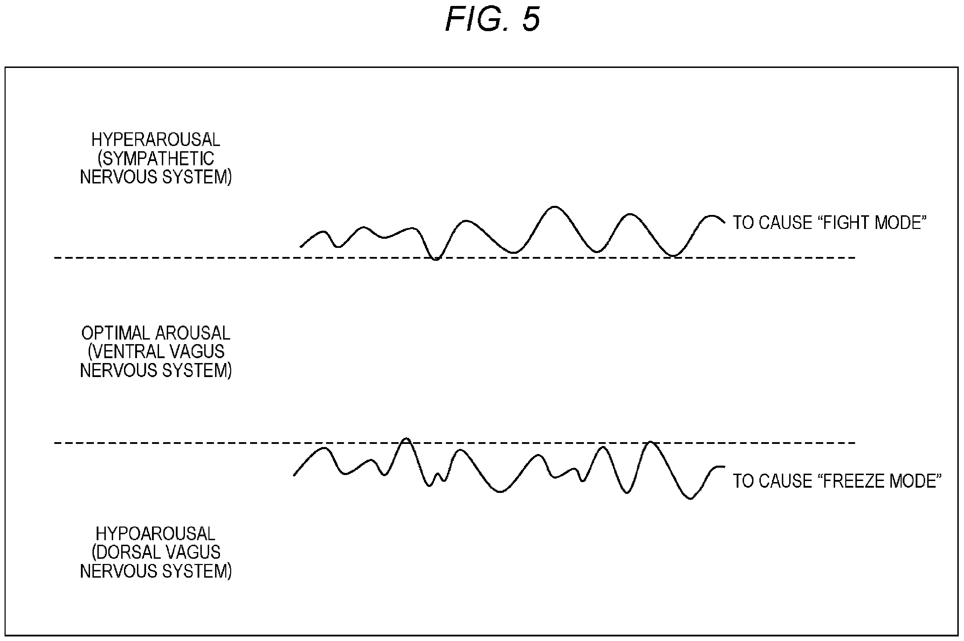

Furthermore, in the example of Fig. 5, a state of a "freeze mode" is continued by the dorsal vagus nerve being strengthened, or a state is of the "fight mode" continued by the sympathetic nerve being strengthened. In the technology of the present disclosure, as in the example of Fig. 5, in a case where the arousal state continues to remain in hypoarousal or hyperarousal, improvement feedback is presented such that the fluctuation of nerve activity shifts to the optimal arousal state (the window of tolerance).

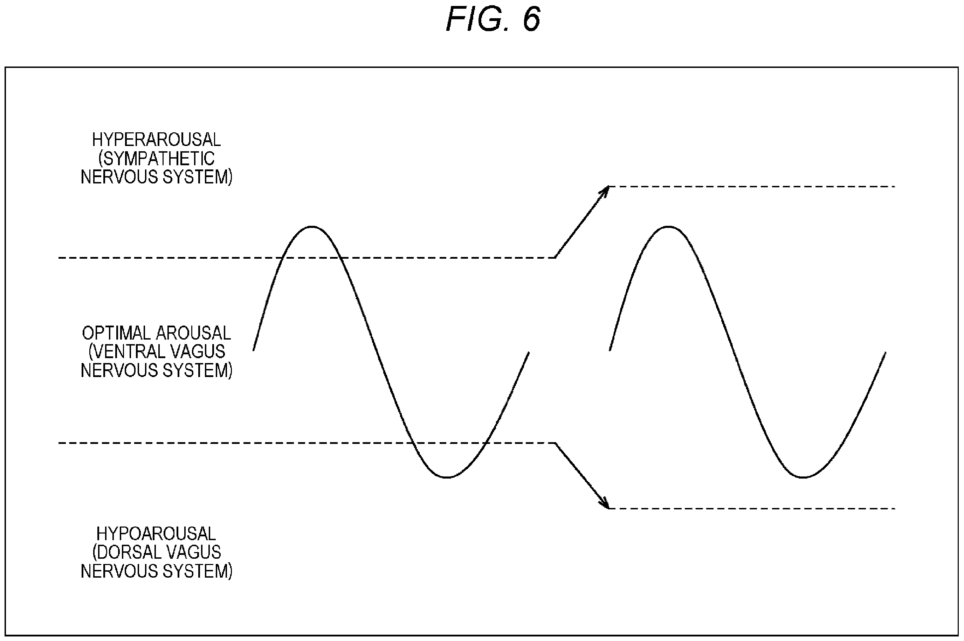

Moreover, in the technology of the present disclosure, as illustrated in Fig. 6, improvement feedback is presented such that the fluctuation of the nerve activity falls within the optimal arousal state (the window of tolerance) by expanding the optimal arousal state (the window of tolerance).

Hereinafter, embodiments of the present disclosure will be described.

### <3. First embodiment of present disclosure>

### (Functional configuration example of information processing apparatus)

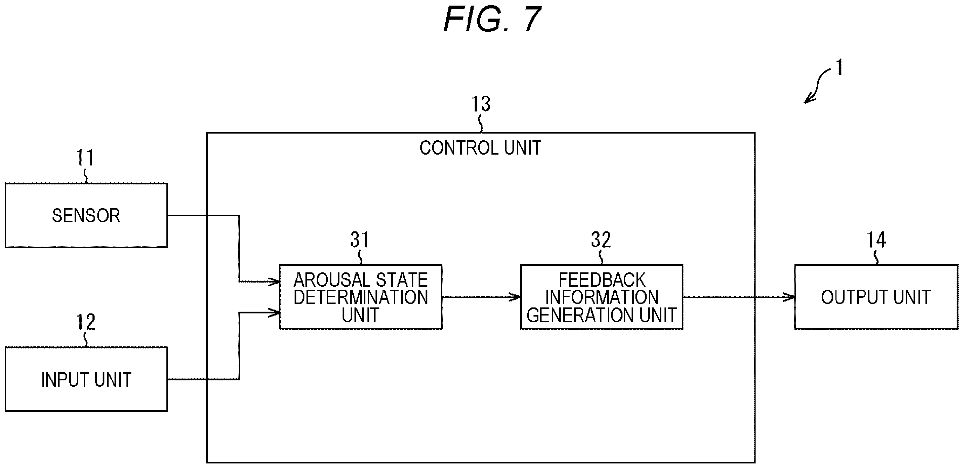

Fig. 7 is a diagram illustrating a functional configuration example of an information processing apparatus according to a first embodiment of the present disclosure.

An information processing apparatus 1 in Fig. 7 is configured as a smartphone held by a hand of a user to which feedback is to be presented, a wearable device worn on a part of the body of the user, a display or a television receiver placed around or in the vicinity of the user, or the like. The wearable device includes a headphone type device, an earphone type device, and the like in addition to a watch type device, a jacket type device, a belt type device, a collar type device, and a glasses type device.

The information processing apparatus 1 includes a sensor 11, an input unit 12, a control unit 13, and an output unit 14.

The sensor 11 includes various sensors that sense a user. The sensor 11 includes individual sensors capable of sensing at least each of user's utterance voice, posture, line-of-sight, expression, and vital signs. That is, the sensor 11 includes a sound sensor, a gyro sensor, a camera, a vital sensor, and the like. A sensing result obtained by the sensor 11 is supplied to the control unit 13.

The input unit 12 includes a button, a key, a touch panel, and the like that receive an input operation by the user. The input unit 12 supplies input information corresponding to the input operation by the user, to the control unit 13.

The control unit 13 includes a processor such as a central processing unit (CPU), and controls each unit of the information processing apparatus 1. The control unit 13 generates image information, text information, audio information, and the like as feedback information for the output unit 14 to present feedback on the basis of the sensing result from the sensor 11 and the input information from the input unit 12, and supplies to the output unit 14.

The output unit 14 includes a display, a speaker, and the like that present feedback to the user. The output unit 14 presents feedback to the user by an image, text, audio, or the like on the basis of the feedback information from the control unit 13. The output unit 14 may include a medical electronic device or the like capable of improving vital signs of the user.

The control unit 13 includes an arousal state determination unit 31 and a feedback information generation unit 32.

The arousal state determination unit 31 determines the arousal state of the user on the basis of the sensing result from the sensor 11 and the input information from the input unit 12. Specifically, the arousal state determination unit 31 determines which state among hyperarousal, optimal arousal, and hypoarousal, in the Polyvagal theory described above, the arousal state of the user is in.

The arousal state determination unit 31 determines the arousal state of the user in time series at predetermined time intervals, and accumulates a determination result in a database (DB) (not illustrated).

The feedback information generation unit 32 generates feedback information for the user, on the basis of the arousal state of the user determined by the arousal state determination unit 31. Specifically, the feedback information generation unit 32 generates feedback information on the basis of determination results of the arousal state of the user accumulated in the DB in a predetermined period, and supplies the feedback information to the output unit 14.

Next, an operation of the information processing apparatus 1 of Fig. 7 will be described.

### (Flow of arousal state determination process)

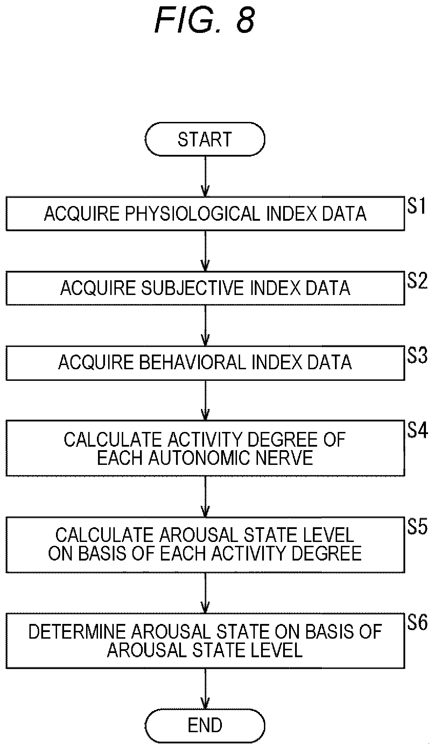

With reference to the flowchart of Fig. 8, a flow of an arousal state determination process by the information processing apparatus 1 of Fig. 7 will be described.

In step S1, the arousal state determination unit 31 acquires physiological index data of the user on the basis of a sensing result from the sensor 11. The physiological index data includes at least any of a sensing result of each of user's voice (utterance voice), posture, line-of-sight, brain waves, saliva components, hormones, and vital signs. The vital signs include, for example, the user's respiration, heartbeat, myoelectricity, perspiration, blood flow, and the like.

In step S2, the arousal state determination unit 31 acquires subjective index data of the user on the basis of input information from the input unit 12. The subjective index data is data personalized to the user, and includes at least any of a personality analysis result and a psychological test result for the user. The subjective index data is acquired, for example, by supplying, from the input unit 12, answers to a questionnaire to the user or the like as input information.

In step S3, the arousal state determination unit 31 acquires behavioral index data of the user on the basis of a sensing result from the sensor 11. The behavioral index data includes at least any of an analysis result of each of a user's motion, expression, line-of-sight, and utterance content.

In step S4, the arousal state determination unit 31 calculates the activity degree of each autonomic nerve (the dorsal vagus nerve, the sympathetic nerve, and the ventral vagus nerve) on the basis of the acquired physiological index data, subjective index data, and behavioral index data. The activity degree of each autonomic nerve is a value obtained by scoring at least any of the physiological index data, the subjective index data, and the behavioral index data of the user.

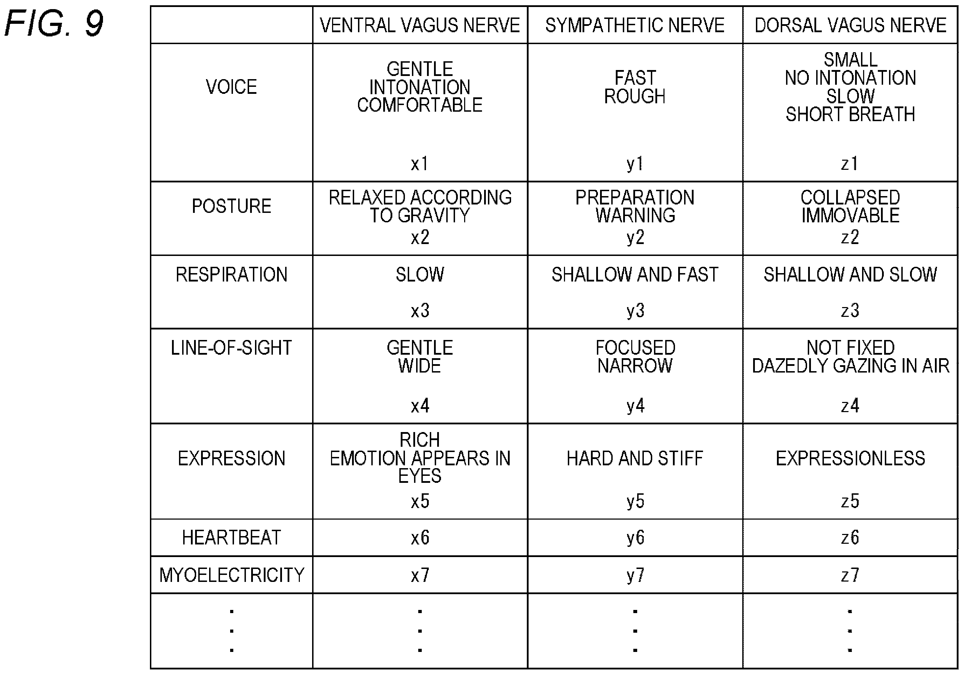

Fig. 9 is a table illustrating an example of the physiological index data. In the example of Fig. 9, voice, a posture, respiration, a line-of-sight, expression, heartbeat, and myoelectricity are illustrated as the physiological index data.

The user's voice is scored with x1, y1, and z1. In a case where the user's voice is gentle, has intonation, or is comfortable, the score x1 for the ventral vagus nerve becomes high. In a case where the user's voice is fast or rough, the value of the score y1 for the sympathetic nerve becomes high. In a case where the user's voice is small, has no intonation, is slow, or has short breath, the value of the score z1 for the dorsal vagus nerve becomes high.

The user's posture is scored with x2, y2, and z2. In a case where the user's posture is relaxed according to gravity, the value of the score x2 for the ventral vagus nerve becomes high. In a case where the user's posture is preparation or warning, the value of the score y2 for the sympathetic nerve becomes high. In a case where the user's posture is collapsed or immovable, the value of the score z2 for the dorsal vagus nerve becomes high.

The user's respiration is scored with x3, y3, and z3. In a case where the user's respiration is slow, the score x3 for the ventral vagus nerve becomes high. In a case where the user's respiration is shallow and fast, the value of the score y3 for the sympathetic nerve becomes high. In a case where the user's respiration is shallow and slow, the value of score z3 for the dorsal vagus nerve becomes high.

The user's line-of-sight is scored with x4, y4, and z4. In a case where the user's line-of-sight is gentle or wide, the value of the score x4 for the ventral vagus nerve becomes high. In a case where the user's line-of-sight is focused or narrow, the value of the score y4 for the sympathetic nerve becomes high. In a case where the user's line-of-sight is not fixed or the user is dazedly gazing in the air, the value of the score z4 for the dorsal vagus nerve becomes high.

The user's expression is scored with x5, y5, and z5. In a case where the user's expression is rich or emotion appears in the eyes, the value of the score x5 for the ventral vagus nerve becomes high. In a case where the user's expression is hard and stiff, the value of the score y5 for the sympathetic nerve becomes high. In a case where the user's expression is expressionless, the value of the score z5 for the dorsal vagus nerve becomes high.

Similarly, the user's heartbeat is scored with x6, y6, and z6, and the user's myoelectricity is scored with x7, y7, and z7.

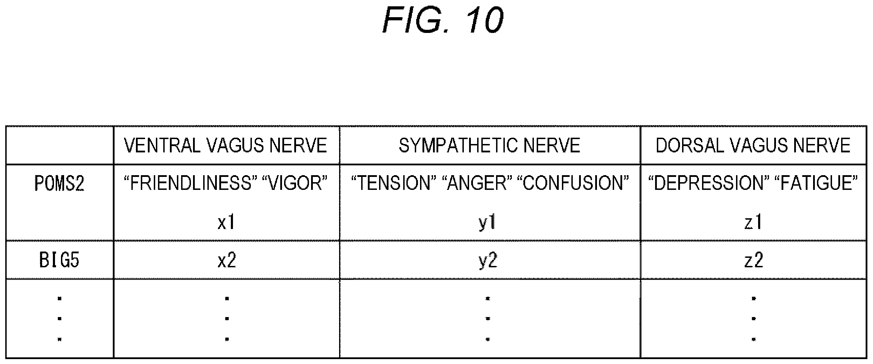

Fig. 10 is a table illustrating an example of the subjective index data. In the example of Fig. 10, the Profile of Mood States (POMS2) and the Big-five factor structure (BIG 5) are illustrated as the subjective index data.

The POMS2 is one of psychological tests that can evaluate a mood state of a user. In the POMS2, the user's mood state is evaluated by seven scales of "tension", "depression", "anger", "vigor", "fatigue", "confusion", and "friendliness".

The user's mood state evaluated by the POMS2 is scored with x1, y1, and z1. In a case where "friendliness" and" vigor" are evaluated to be high for the user's mood state, the value of the score x1 for the ventral vagus nerve becomes high. In a case where "tension", "anger", and "confusion" are evaluated to be high for the user's mood state, the value of the score y1 for the sympathetic nerve becomes high. In a case where "depression" and "fatigue" are evaluated to be high for the user's mood state, the value of the score z1 for the dorsal vagus nerve becomes high.

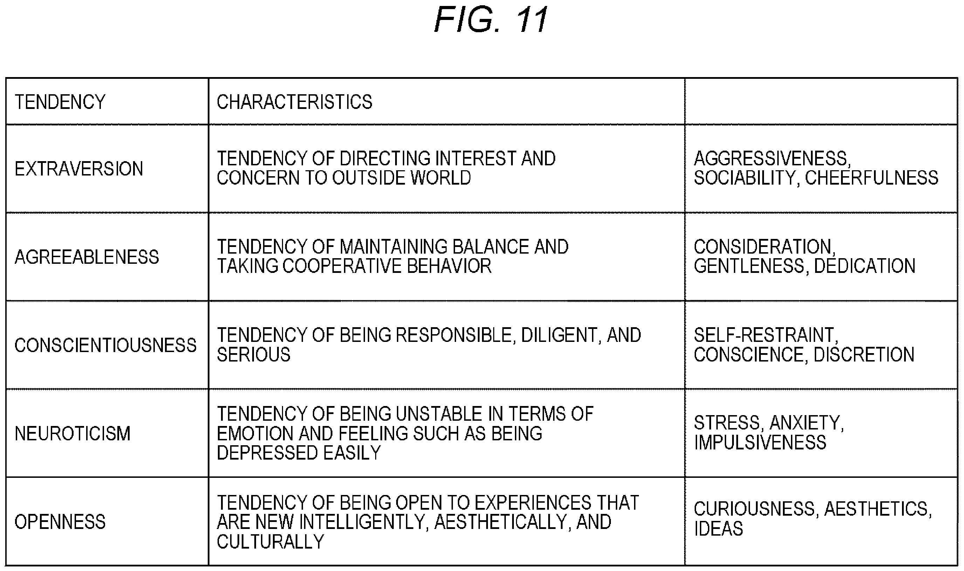

The BIG 5 is one of character analysis theories. As illustrated in Fig. 11, in the BIG 5, the user's personality is analyzed by a combination of five personality factors (tendencies) of "extraversion", "agreeableness", "conscientiousness", "neuroticism", and "openness".

Characteristics of "extraversion" have a tendency of directing interest and concern to the outside world, and aggressiveness, sociability, cheerfulness, and the like greatly act. Characteristics of "agreeableness" have a tendency of maintaining balance and taking a cooperative behavior, and consideration, gentleness, dedication, and the like greatly act. Characteristics of "conscientiousness" have a tendency of being responsible, diligent, and serious, and self-restraint, conscience, discretion, and the like greatly act. Characteristics of the "neuroticism" have a tendency of being unstable in terms of emotion and feeling such as being depressed easily, and stress, anxiety, impulsiveness, and the like greatly act. Characteristics of "openness" have a tendency of being open to experiences that are new intelligently, aesthetically, and culturally, and curiousness, aesthetics, ideas, and the like greatly act.

Then, in the subjective index data, the user's personality analyzed by the BIG 5 as described above can also be scored with x2, y2, and z2.

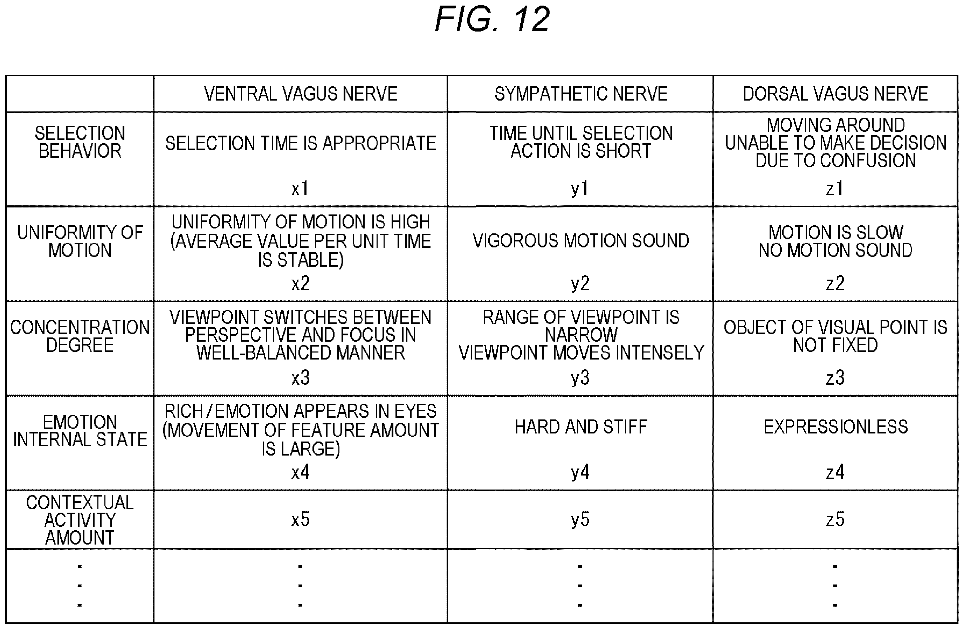

Fig. 12 is a table illustrating an example of the behavioral index data. In the example of Fig. 12, selection behavior, uniformity of motion, a concentration degree, a contextual activity amount, and an emotion internal state are illustrated as the behavioral index data. These pieces of the behavioral index data are acquired not as immediate data but as analysis logs of sensing results for a certain period of time. Furthermore, a change in a daily behavior (routine) of the user may be acquired as the behavioral index data.

The selection behavior is scored with x1, y1, and z1. In a case where selection time is appropriate as the selection behavior, the value of the score x1 for the ventral vagus nerve becomes high. In a case where time until a selection action is short as the selection behavior, the value of the score y1 for the sympathetic nerve becomes high. In a case of moving around or being unable to make a decision due to confusion as the selection behavior, the value of the score z1 for the dorsal vagus nerve becomes high.

The uniformity of motion is scored with x2, y2, and z2. In a case where the uniformity of motion is high (an average value per unit time is stable), the value of score x2 for the ventral vagus nerve becomes high. In a case where vigorous motion sound is accompanied in the motion, the value of the score y2 for the sympathetic nerve becomes high. In a case where the motion is slow or there is no motion sound, the value of the score z2 for the dorsal vagus nerve becomes high.

The concentration degree is scored with x3, y3, and z3. In a case where a viewpoint switches between perspective and focus in a well-balanced manner, the value of the score x3 for the ventral vagus nerve becomes high. In a case where a range of the viewpoint is narrow or the viewpoint moves intensely, the value of the score y3 for the sympathetic nerve becomes high. In a case where an object of the visual point is not fixed, the value of the score z3 for the dorsal vagus nerve becomes high.

The emotion internal state is scored with x4, y4, and z4. In a case where expression is rich and emotion appears in the eyes (a movement of a feature amount is large), the value of the score x5 for the ventral vagus nerve becomes high. In a case where expression is hard and stiff, the value of the score y5 for the sympathetic nerve becomes high. In the case of being expressionless, the value of the score z5 for the dorsal vagus nerve becomes high. Note that the feature amount here is, for example, facial expression, a motion analysis result by skeleton estimation, a line-of-sight estimation result, an utterance content, motion sound, or the like.

Similarly, the contextual activity amount is scored with x5, y5, and z5.

As described above, the physiological index data, the subjective index data, and the behavioral index data of the user are scored.

Then, an activity degree X of the ventral vagus nerve is calculated by X = f1(x1, x2, x3, x4, x5, ···). An activity degree Y of the sympathetic nerve is calculated by Y = f2(y1, y2, y3, y4, y5, ···). An activity degree Z of the dorsal vagus nerve is calculated by Z = f3(z1, z2, z3, z4, z5, ···). For example, f1 is a function that takes a value closer to 0 as a parameter takes a larger value. For example, f2 is a function that takes a larger value as a parameter takes a larger value. For example, f3 is a function that takes a negative value having a larger absolute value as a parameter takes a larger value.

Here, the scores x1, x2, x3, x4, x5, ···, y1, y2, y3, y4, y5, ···, z1, z2, z3, z4, z5, ··· as parameters of each function are values obtained by integrating individual values obtained by scoring the individual items of the physiological index data, the subjective index data, and the behavioral index data, for example.

Returning to the flowchart of Fig. 8, in step S5, the arousal state determination unit 31 calculates an arousal state level indicating the arousal state of the user, on the basis of each of the activity degrees X, Y, and Z. The arousal state level is calculated at predetermined time intervals.

The arousal state level is, for example, obtained as the sum of: an optimal arousal score Xa calculated using the activity degree X of the ventral vagus nerve; a hyperarousal score Yb calculated using the activity degree Y of the sympathetic nerve; and a hypoarousal score Zc calculated using the activity degree Z of the dorsal vagus nerve.

For example, the optimal arousal score Xa is a value obtained by multiplying the activity degree X of the ventral vagus nerve by a coefficient a1 and adding an offset value a2. The hyperarousal score Yb is a value obtained by multiplying the activity degree Y of the sympathetic nerve by a coefficient b1 and adding an offset value b2. The hypoarousal score Zc is a value obtained by multiplying the activity degree Z of the dorsal vagus nerve by a coefficient c1 and adding an offset value c2. Each coefficient and each offset value are set for each user, or are varied according to an accumulation result of the arousal state of the user.

Then, in step S6, the arousal state determination unit 31 determines the arousal state on the basis of the calculated arousal state level. The determination result of the arousal state is accumulated in a DB (not illustrated) in association with the arousal state level as a label at the time when the arousal state level is calculated.

In the present embodiment, feedback to the user is presented on the basis of the arousal state determined in this manner.

### (Flow of feedback presentation process)

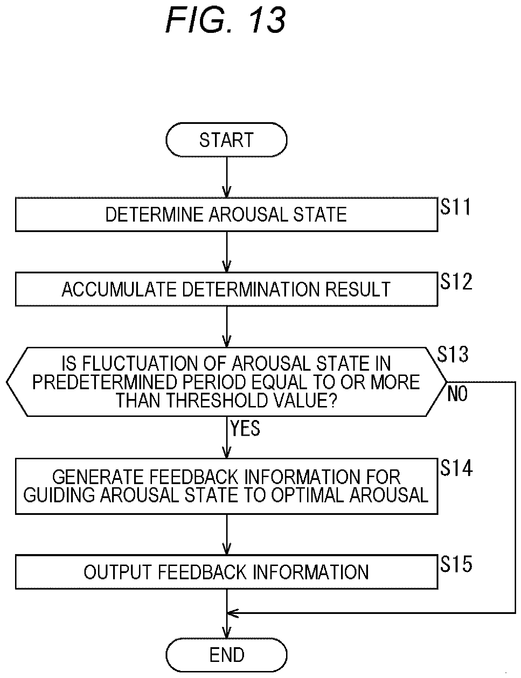

Next, with reference to a flowchart of Fig. 13, a flow of a feedback presentation process by the information processing apparatus 1 of Fig. 7 will be described.

In step S11, the arousal state determination unit 31 determines the arousal state of the user on the basis of a sensing result from the sensor 11 and input information from the input unit 12. Here, the arousal state determination process described with reference to Fig. 8 is executed at predetermined time intervals.

In step S12, the arousal state determination unit 31 accumulates a determination result of the arousal state in a DB (not illustrated).

In step S13, on the basis of the determination result of the arousal state accumulated in the DB (not illustrated), the feedback information generation unit 32 determines whether or not a fluctuation of the arousal state in a predetermined period is equal to or more than a threshold value. The fluctuation of the arousal state in the predetermined period includes, for example, a staying status in hyperarousal or hypoarousal and a transition frequency between hyperarousal and hypoarousal, of the arousal state in the predetermined period.

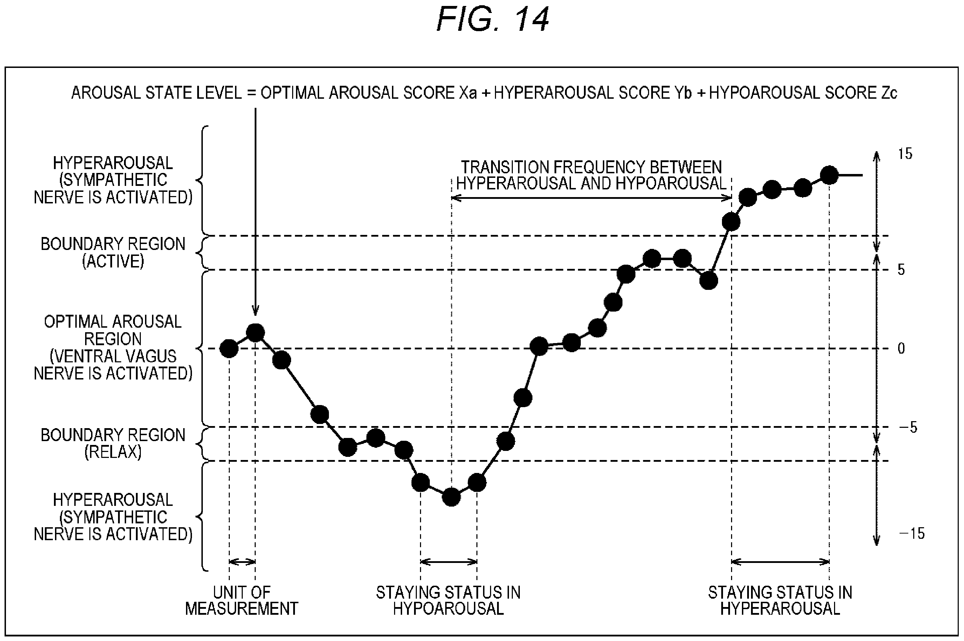

Fig. 14 is a view illustrating an example of a fluctuation of an arousal state.

In Fig. 14, an arousal state level calculated with a horizontal direction as a time direction and a predetermined time interval as a unit of measurement is plotted. The unit of measurement is, for example, about one minute to five minutes. In Fig. 14, a vertical direction indicates magnitude of a score of the arousal state level, and the score of the arousal state level changes in a range of -15 to 15 with 0 as a center, in the example of Fig. 14.

In a case where the arousal state level is in a range of approximately -5 to 5, the arousal state level at that time is determined to be optimal arousal (the ventral vagus nerve is activated). In a case where the arousal state level is below -5, the arousal state level at that time is determined to be hypoarousal (the dorsal vagus nerve is activated). In a case where the arousal state level exceeds 5, the arousal state level at that time is determined to be hyperarousal (the sympathetic nerve is activated).

Note that the vicinity of the arousal state level of -5 is regarded as a boundary region between optimal arousal and hypoarousal. In this boundary region, since there is a high possibility that the person is in a relaxed state, the arousal state may be determined as optimal arousal. Furthermore, the vicinity of the arousal state level of 5 is regarded as a boundary region between optimal arousal and hyperarousal. In this boundary region, since there is a high possibility that the person is in a moderately active state, the arousal state may be determined as optimal arousal.

In the example of Fig. 14, as a staying status in hypoarousal, the arousal state level remains in a range of hypoarousal for a length of time of at least two units of measurement at some point of the predetermined period. Furthermore, as a staying status in hyperarousal, the arousal state level remains in a range of hyperarousal for a length of time of at least four units of measurement at some point of the predetermined period.

In step S13, it is determined whether or not a certain degree is exceeded by a degree to which the arousal state level remains in a range of hypoarousal or hyperarousal (the staying status). Specifically, for example, it is determined whether or not a value based on magnitude of an extreme value of the arousal state level and a multiplication value for that period while the arousal state level remains in a range of hypoarousal or hyperarousal is greater than a predetermined threshold value. Furthermore, it is determined whether or not an integral value while the arousal state level remains in a range of hypoarousal or hyperarousal exceeds a predetermined threshold value.

Furthermore, in the example of Fig. 14, as a transition frequency between hyperarousal and hypoarousal, the arousal state level transitions from hypoarousal to hyperarousal over time of at least 12 units of measurement at some point of the predetermined period.

In step S13, it is determined whether or not the number of times of transitions (the transition frequency) of the arousal state level from hypoarousal to hyperarousal (or from hyperarousal to hypoarousal) exceeds a certain number of times within a certain period of time.

Then, when it is determined in step S13 that the fluctuation of the arousal state in the predetermined period is equal to or more than the threshold value, that is, when the staying status in hyperarousal or hypoarousal exceeds a certain level, or when the transition frequency between hyperarousal and hypoarousal exceeds a certain number of times, the process proceeds to step S14.

In step S14, the feedback information generation unit 32 generates feedback information for guiding the arousal state to optimal arousal.

Thereafter, in step S15, the output unit 14 presents feedback to the user by outputting the generated feedback information. Note that, when it is determined in step S13 that the fluctuation of the arousal state in the predetermined period is not equal to or more than the threshold value, steps S14 and S15 are skipped, and the process ends.

Here, feedback information for guiding the arousal state to optimal arousal is generated and presented in accordance with a short-term arousal state.

For example, in order to guide the arousal state from hyperarousal to optimal arousal, it is necessary to present feedback for activating the ventral vagus nerve and assisting the dorsal vagus nerve.

Specifically, in a case where the output unit 14 includes a medical electronic device or the like capable of improving vital signs of the user, feedback for bringing heartbeat into a coherent state or feedback for realizing acoustic therapy is presented.

Moreover, feedback suggesting deep breathing, mediation, or the like as exercise, or feedback suggesting refraining from stimulants such as coffee as a behavior, for example, may be presented by an image, text, audio, or the like.

Furthermore, in order to guide the arousal state from hypoarousal to optimal arousal, it is necessary to present feedback for activating the ventral vagus nerve and assisting the sympathetic nerve.

Specifically, in a case where the output unit 14 includes a medical electronic device or the like capable of improving vital signs of the user, feedback for realizing vagus nerve stimulation therapy is presented.

Moreover, feedback suggesting performing light exercise, yoga, or the like as exercise or feedback suggesting talking with a close friend as a behavior, for example, may be presented by an image, text, audio, or the like.

Note that, in a case where the arousal state is in hyperarousal or hypoarousal for a long time, as feedback for guiding the arousal state to optimal arousal, for example, feedback suggesting reception of therapy or medical action is presented by an image, text, audio, or the like.

According to the above process, it is determined which state among hyperarousal, optimal arousal, and hypoarousal, in the Polyvagal theory, the arousal state of the user is in. That is, since the feedback information is generated on the basis of the objective mental state index, more appropriate feedback can be presented.

### <4. Second embodiment of present disclosure>

### (Functional configuration example of information processing apparatus)

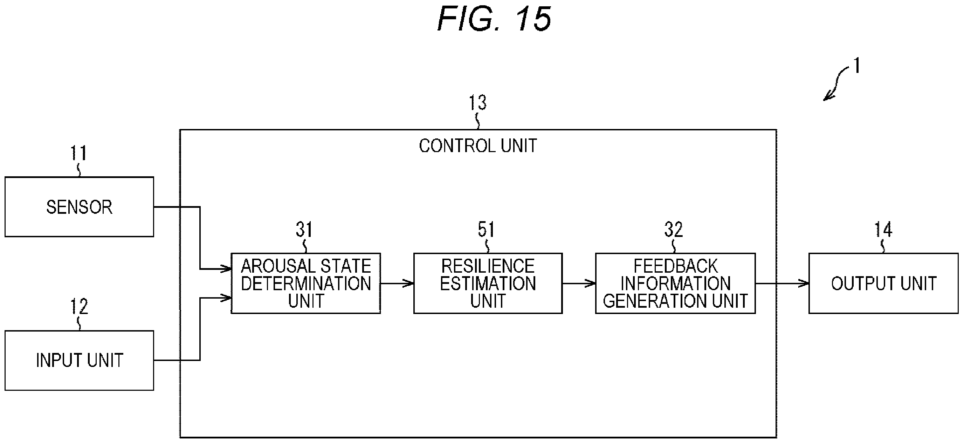

Fig. 15 is a diagram illustrating a functional configuration example of an information processing apparatus according to a second embodiment of the present disclosure.

An information processing apparatus 1 of Fig. 15 is basically configured similarly to the information processing apparatus 1 of Fig. 7, but is different in that a control unit 13 includes a resilience estimation unit 51 in addition to an arousal state determination unit 31 and a feedback information generation unit 32.

The resilience estimation unit 51 estimates strength of resilience of a user on the basis of an arousal state of the user determined by the arousal state determination unit 31. Specifically, the resilience estimation unit 51 estimates the strength of resilience of the user on the basis of a determination result of the arousal state of the user accumulated in a DB for a predetermined period, and supplies the estimation result to the feedback information generation unit 32.

The term "resilience" as used herein refers to a force with which a person tries to live flexibly according to a situation without giving up even in a difficult or adverse situation. In the present embodiment, the strength of resilience is defined by a width of a range of "optimal arousal" (the window of tolerance).

The feedback information generation unit 32 in Fig. 15 generates feedback information on the basis of the estimated strength of resilience, and supplies the feedback information to an output unit 14.

Next, an operation of the information processing apparatus 1 of Fig. 15 will be described.

### (Feedback presentation process)

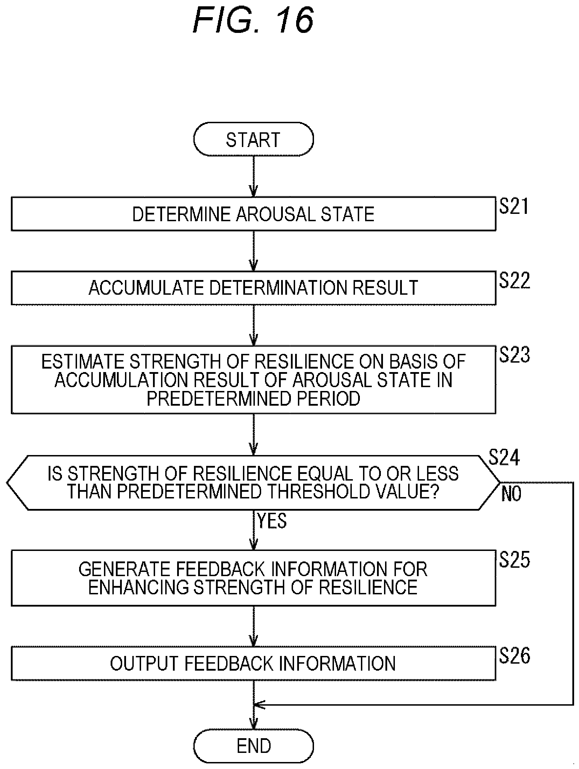

With reference to the flowchart in Fig. 16, a feedback presentation process by the information processing apparatus 1 in Fig. 15 will be described. Note that the processing of steps S21 and S22 in the flowchart of Fig. 16 is similar to the processing of steps S11 and S12 in the flowchart of Fig. 13, and thus the description thereof will be omitted.

That is, in step S23, the resilience estimation unit 51 estimates the strength of resilience of the user on the basis of accumulation results of the arousal state accumulated in a DB in a predetermined period.

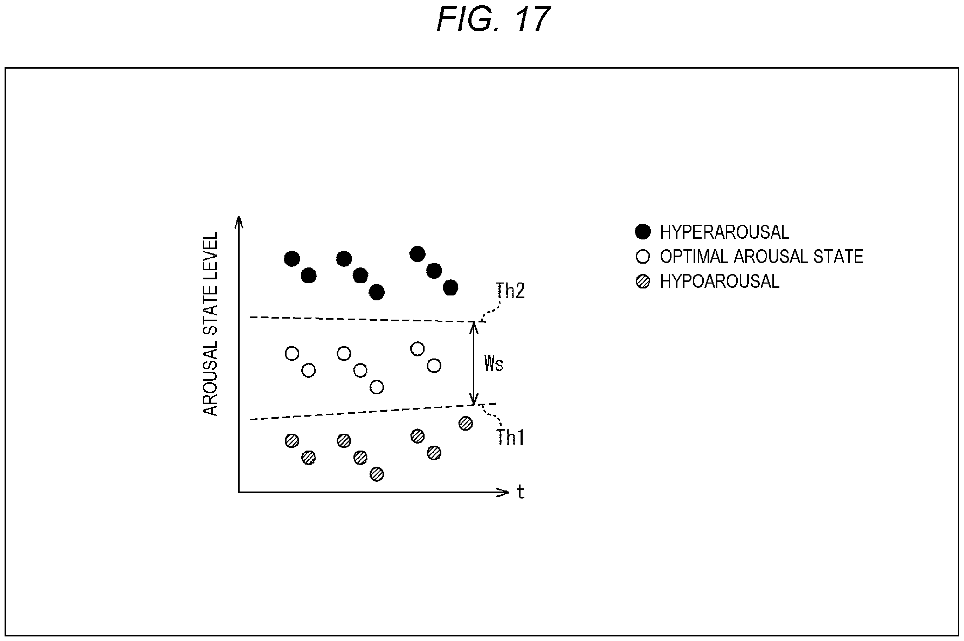

For example, as illustrated in Fig. 17, it is assumed that an arousal state level is calculated (measured) in a predetermined period. In the example of Fig. 17, a plot indicating an arousal state level on a time axis is illustrated for each label associated with the arousal state level. Specifically, an arousal state level indicated by a black plot is associated with a label of hyperarousal, and an arousal state level indicated by a white plot is associated with a label of optimal arousal. The arousal state level indicated by a hatched plot is associated with a label of hypoarousal.

The resilience estimation unit 51 calculates a threshold value for determination of the arousal state, for the arousal state level for a predetermined period as illustrated in Fig. 17. In the example of Fig. 17, a threshold value Th1 for hypoarousal and optimal arousal and a threshold value Th2 for hyperarousal and optimal arousal are calculated. The threshold values Th1 and Th2 are obtained as boundaries for classifying the arousal state level into three classes of hyperarousal, optimal arousal, and hypoarousal, for example, by support vector machines (SVM). Then, a width Ws between the threshold value Th1 and the threshold value Th2 is defined as the strength of resilience.

Returning to the flowchart of Fig. 16, in step S24, the feedback information generation unit 32 determines whether or not the strength of resilience (the width Ws) is equal to or less than a predetermined threshold value Wth. When it is determined that the strength of resilience (the width Ws) is equal to or less than the threshold value Wth, the process proceeds to step S25.

In step S25, the feedback information generation unit 32 generates feedback information for enhancing the strength of resilience.

Thereafter, in step S26, the output unit 14 presents feedback to the user by outputting the generated feedback information. Note that, when it is determined in step S24 that the strength of resilience (the width Ws) is not equal to or less than the threshold value Wth, steps S25 and S26 are skipped, and the process ends.

Here, feedback information for enhancing the strength of resilience is generated and presented according to a short-term arousal state.

In order to improve the strength of resilience, it is necessary to present feedback for expanding the window of tolerance.

Specifically, in a case where the output unit 14 includes a medical electronic device or the like capable of improving vital signs of the user, feedback for realizing acoustic therapy is presented. As the acoustic therapy here, for example, a method using a hypersonic effect, the Tomatis method, a safe and sound protocol (SSP), and the like are applied.

Furthermore, feedback for realizing vagus nerve stimulation therapy or percutaneous vibration acoustic stimulation therapy may be presented, or feedback for bringing heartbeat into a coherent state by the HeartMath method or the like may be presented.

Moreover, feedback information suggesting, as exercise, performing a face exercise of moving facial expression muscles of the face, yoga, karaoke, or mental fullness mediation may be presented by an image, text, audio, or the like.

Note that, in a case where the arousal state is in hyperarousal or hypoarousal for a long time, as feedback for enhancing the strength of resilience, for example, feedback suggesting reception of therapy or medical response is presented by an image, text, audio, or the like.

According to the above processing, the width of the window of tolerance, which is a range in which stress can be tolerated, is estimated on the basis of the arousal state of the user. That is, since the feedback information based on the objective mental state index is generated, more appropriate feedback can be presented.

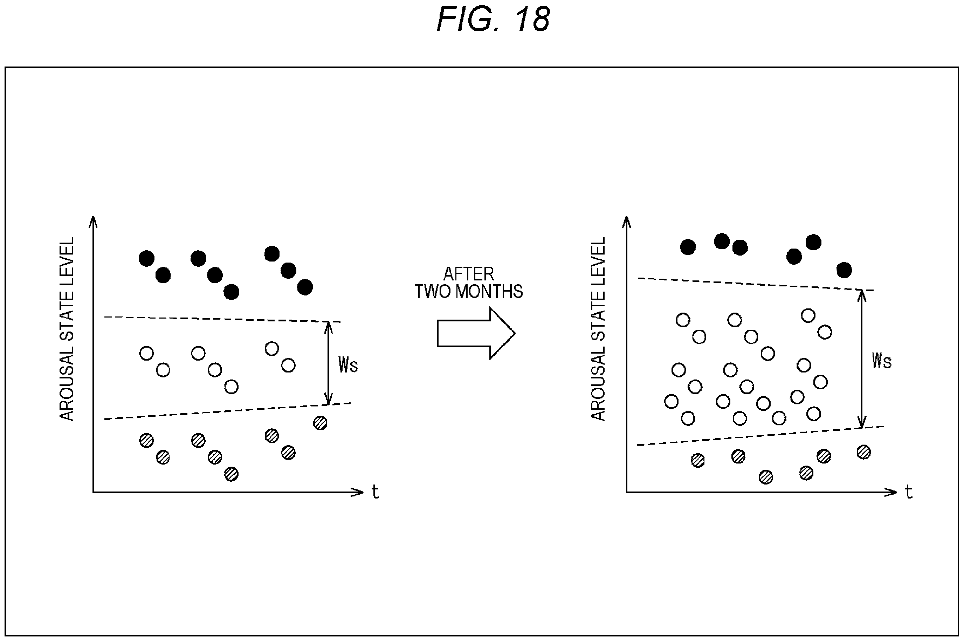

Note that an image representing the strength of resilience (the width Ws of the window of tolerance) as exemplified in Fig. 17 may be presented as feedback. In this case, as illustrated in Fig. 18, the user can visually understand that the width Ws of the window of tolerance increases, for example, after two months. That is, a degree of improvement in a mental state can be objectively grasped, and long-term motivation of the user can be improved.

In the above description, the strength of resilience is estimated from the threshold value of the classification of the arousal state level (the arousal state) on the time axis, but the strength of resilience may be estimated from a distribution status of the arousal state as an accumulation result in a predetermined period.

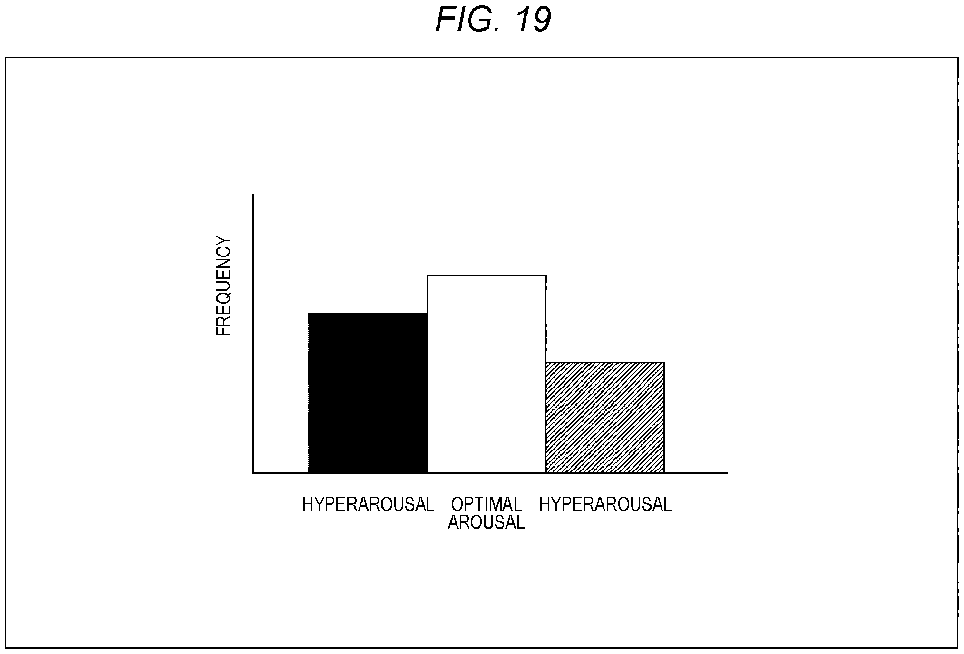

For example, as illustrated in Fig. 19, it is assumed that a distribution of arousal states in a predetermined period is expressed by a histogram. In this case, a frequency of optimal arousal may be set as the strength of resilience, and it may be determined whether or not the strength of resilience (the frequency of optimal arousal) is equal to or less than a predetermined threshold value.

### (Another example of feedback presentation process)

In the above description, improvement feedback for making the arousal state fall within optimal arousal and improvement feedback for widening the window of tolerance have been presented according to the arousal state and the strength of resilience. Without limiting to this, for example, as exemplified in Fig. 18, in a case where the strength of resilience is continuously improved with the lapse of time, feedback for maintaining a favorable arousal state may be presented.

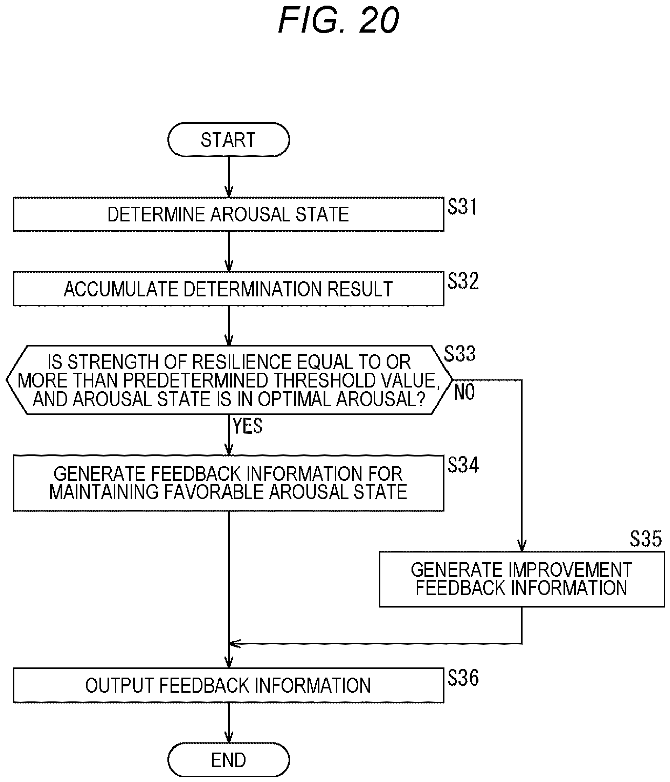

Therefore, with reference to the flowchart of Fig. 20, a feedback presentation process for maintaining a favorable arousal state by the information processing apparatus 1 of Fig. 15 will be described. Note that the processing of steps S31 and S32 in the flowchart of Fig. 20 is similar to the processing of steps S11 and S12 in the flowchart of Fig. 13, and thus the description thereof will be omitted.

That is, in step S33, the feedback information generation unit 32 determines whether or not the strength of resilience (the width Ws of the window of tolerance) in a predetermined period is equal to or more than the predetermined threshold value Wth, and the arousal state is in optimal arousal. When it is determined that the strength of resilience (the width Ws of the window of tolerance) is equal to or more than the predetermined threshold value Wth and the arousal state is in optimal arousal, the process proceeds to step S34.

In step S34, the feedback information generation unit 32 generates feedback information for maintaining the favorable arousal state.

Whereas, in a case where it is determined in step S33 that the strength of resilience (the width Ws of the window of tolerance) is not equal to or more than the predetermined threshold value Wth or the arousal state is not in optimal arousal, the process proceeds to step S35.

In step S35, the feedback information generation unit 32 generates improvement feedback information. That is, the feedback information is generated that is for presenting the improvement feedback for making the arousal state fall within optimal arousal or the improvement feedback for widening the window of tolerance.

After step S34 or step S35, in step S36, the output unit 14 presents feedback to the user by outputting the generated feedback information.

According to the above processing, in a case where the arousal state of the user is in a favorable state, the favorable arousal state can be maintained.

### <5. Use case>

The technology according to the present disclosure can be applied to the following use cases.

### (Use case 1: Kitchen)

In a case where the user standing in the kitchen is recognized by behavior recognition based on a sensing result, the user's voice, posture, respiration, line-of-sight, expression, heartbeat, skin state, blood flow, and the like are sensed, with the behavior recognition as a trigger. These may be constantly sensed.

In the kitchen, in a case where it is determined that thinking time of menu by the user is longer than usual, a speed at which the user cuts vegetables is slower than usual, or the like, improvement feedback to the user is presented. This improvement feedback is presented to one person in the kitchen. For example, as the improvement feedback, a message such as "You seem to feel excessive stress now. Sing to relieve stress!" is presented by text or audio. After such a message is presented, the user's favorite music may be output.

### (Use case 2: Meeting room)

In a case where behavior recognition based on a sensing result recognizes the user having entered a conference room in which a conference is to be held, the user's voice, posture, respiration, line-of-sight, expression, heartbeat, skin state, blood flow, and the like are sensed, with the behavior recognition as a trigger. These may be constantly sensed.

In a case where it is determined that time required for determining a policy is longer than usual in the conference room, or the like, improvement feedback to the user is presented. This improvement feedback is presented not only to one person but also to all the persons participating in the meeting. For example, as the improvement feedback, a message such as "Concentration of each is insufficient. How about having a coffee break?" is presented by text or audio. Furthermore, as the improvement feedback, feedback suggesting loosening muscles around the eyes and the shoulders or repeating bending and stretching of the fingers may be presented by an image, text, audio, or the like.

In the use cases described above, a long-term arousal state of the user may be recognized by machine learning or the like while presentation of the feedback triggered by the behavior recognition is repeated. In this case, feedback corresponding to the recognized long-term arousal state is presented.

For example, in a case where the window of tolerance has expanded over several months, a message such as "You used to be irritated quickly, but now the frequency of irritation is reduced" is presented by text or audio. Furthermore, in a case where the state of being in hyperarousal continues, a message such as "You have a very high rate of the hyperarousal state for three months. It seems better to get medicine at the hospital" is presented by text or audio.

Furthermore, as described above, feedback according to an arousal state of one person may be presented to a plurality of persons, in addition to being presented to the one person. In this case, by presenting the feedback according to the arousal state of one member having a positive influence on a group to other members in the group, motivation of the entire group can be improved.

Furthermore, feedback according to arousal states of a plurality of persons may be presented to a plurality of other persons. In this case, it is possible to improve teamwork of another group by presenting, to the another group, the feedback according to the arousal states of a group having good teamwork.

Moreover, feedback according to arousal states of a plurality of persons may be presented to one person. In this case, by presenting the feedback according to the arousal states of the entire group to one member in the group, a position and a task of the member in the group can be appropriately understood.

### <6. Configuration example of computer>

The above-described series of processing can be executed by hardware or can be executed by software. In a case where the series of processes are performed by software, a program that configures the software is installed in a computer. Here, examples of the computer include, for example, a computer that is built in dedicated hardware, a general-purpose personal computer that can perform various functions by being installed with various programs, and the like.

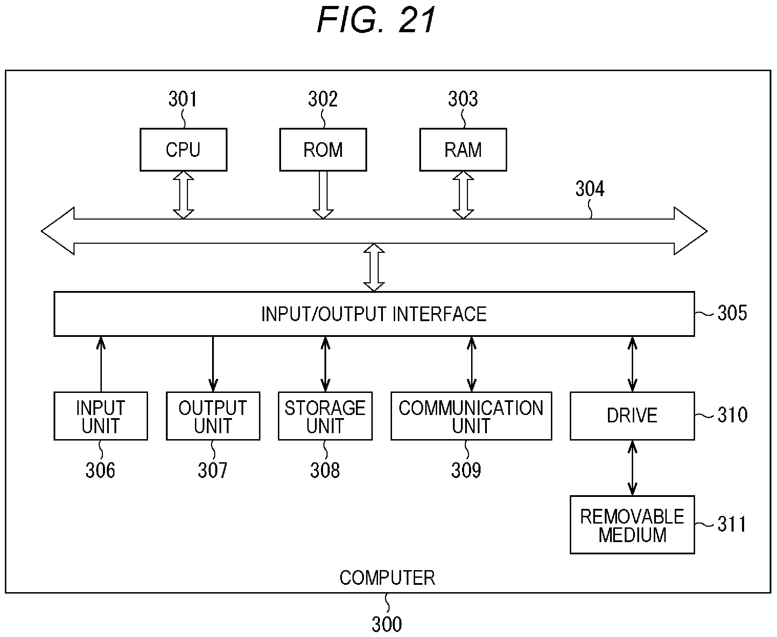

Fig. 21 is a block diagram illustrating a configuration example of hardware of a computer that executes the series of processes described above in accordance with a program.

In a computer 300, a central processing unit (CPU) 301, a read only memory (ROM) 302, and a random access memory (RAM) 303 are mutually connected by a bus 304.

The bus 304 is further connected with an input/output interface 305. To the input/output interface 305, an input unit 306, an output unit 307, a storage unit 308, a communication unit 309, and a drive 310 are connected.

The input unit 306 includes a keyboard, a mouse, a microphone, and the like. The output unit 307 includes a display, a speaker, and the like. The storage unit 308 includes a hard disk, a nonvolatile memory, and the like. The communication unit 309 includes a network interface or the like. The drive 310 drives a removable medium 311 such as a magnetic disk, an optical disk, a magneto-optical disk, or a semiconductor memory.

In the computer 300 configured as described above, the series of processes described above are performed, for example, by the CPU 301 loading a program recorded in the storage unit 308 into the RAM 303 via the input/output interface 305 and the bus 304, and executing.

The program executed by the computer 300 (the CPU 301) can be provided by being recorded on, for example, the removable medium 311 as a package medium or the like. Furthermore, the program can be provided via a wired or wireless transmission medium such as a local area network, the Internet, or digital satellite broadcasting.

In the computer 300, by attaching the removable medium 311 to the drive 310, the program can be installed in the storage unit 308 via the input/output interface 305. Furthermore, the program can be received by the communication unit 309 via a wired or wireless transmission medium, and installed in the storage unit 308. Besides, the program can be installed in advance in the ROM 302 and the storage unit 308.

Note that the program executed by the computer 300 may be a program that performs processing in time series according to an order described in this specification, or may be a program that performs processing in parallel or at necessary timing such as when a call is made.

In this specification, a step of describing the program recorded in the recording medium includes not only the processes chronologically performed in the described order but also the processes executed in parallel or individually, which are not necessarily chronologically performed.

The embodiments of the technology according to the present disclosure are not limited to the above-described embodiments, and various modifications can be made without departing from the scope of the technology according to the present disclosure.

For example, the technology according to the present disclosure can have a cloud computing configuration in which one function is shared and processed in cooperation by a plurality of devices via a network.

Furthermore, each step described in the above-described flowchart can be executed by one device, and also shared and executed by a plurality of devices.

Moreover, in a case where one step includes a plurality of processes, the plurality of processes included in the one step can be executed by one device, and also shared and executed by a plurality of devices.

The effects described in the present description are merely examples and are not limited, and other effects may be provided.

Moreover, the technology according to the present disclosure can have the following configurations.
(1) An information processing apparatus including:
   an arousal state determination unit configured to determine which state among hyperarousal, optimal arousal, and hypoarousal an arousal state of a user is in.
(2) The information processing apparatus according to (1), in which
   the arousal state determination unit determines the arousal state by calculating an arousal state level indicating the arousal state.
(3) The information processing apparatus according to (2), in which
   the arousal state determination unit calculates the arousal state level on the basis of an activity degree of autonomic nerve of the user.
(4) The information processing apparatus according to (3), in which
   the autonomic nerve includes a sympathetic nerve, a ventral vagus nerve, and a dorsal vagus nerve.
(5) The information processing apparatus according to (4), in which
   the activity degree is a value obtained by scoring at least any of physiological index data, subjective index data, and behavioral index data of the user.
(6) The information processing apparatus according to (5), in which
   the physiological index data includes at least any of a sensing result of each of utterance voice, a posture, a line-of-sight, expression, a brain wave, a saliva component, a hormone, and a vital sign of the user.
(7) The information processing apparatus according to (5), in which
   the subjective index data includes at least any of a personality analysis result and a psychological test result for the user.
(8) The information processing apparatus according to (5), in which
   the behavioral index data includes at least any of an analysis result of each of a motion, expression, a line-of-sight, and an utterance content of the user.
(9) The information processing apparatus according to any one of (1) to (8), further including:
   a feedback information generation unit configured to generate feedback information for the user on the basis of the determined arousal state.
(10) The information processing apparatus according to (9), in which
   the arousal state determination unit determines the arousal state in time series, and
   the feedback information generation unit generates the feedback information in accordance with a fluctuation of the arousal state in a predetermined period.
(11) The information processing apparatus according to (10), in which
   the feedback information generation unit generates the feedback information in accordance with a staying status of the arousal state in the hyperarousal or the hypoarousal.
(12) The information processing apparatus according to (10), in which
   the feedback information generation unit generates the feedback information in accordance with a transition frequency of the arousal state between the hyperarousal and the hypoarousal.
(13) The information processing apparatus according to any of (10) to (12), in which
   the feedback information generation unit generates the feedback information for guiding the arousal state of the user to the optimal arousal.
(14) The information processing apparatus according to (9), further including:
   a resilience estimation unit configured to estimate strength of resilience of the user on the basis of the arousal state, in which
   the feedback information generation unit generates the feedback information on the basis of the estimated strength of resilience.
(15) The information processing apparatus according to (14), in which
   the resilience estimation unit estimates the strength of resilience on the basis of an accumulation result of the arousal state in a predetermined period.
(16) The information processing apparatus according to (15), in which
   the resilience estimation unit estimates the strength of resilience from a threshold value of classification of the arousal state as the accumulation result.
(17) The information processing apparatus according to (15), in which
   the resilience estimation unit estimates the strength of resilience from a distribution status of the arousal state as the accumulation result.
(18) The information processing apparatus according to any of (14) to (17), in which
   the feedback information generation unit generates the feedback information for improving the strength of resilience.
(19) An information processing method including
   by an information processing apparatus:
   determining which state among hyperarousal, optimal arousal, and hypoarousal an arousal state of a user is in; and
   generating feedback information for the user on the basis of the determined arousal state.
(20) A program for causing a computer to execute processing including:
   determining which state among hyperarousal, optimal arousal, and hypoarousal an arousal state of a user is in; and
   generating feedback information for the user on the basis of the determined arousal state.

### REFERENCE SIGNS LIST

- 1: Information processing apparatus
- 11: Sensor
- 12: Input unit
- 13: Control unit
- 14: Output unit
- 31: Arousal state determination unit
- 32: Feedback information generation unit
- 51: Resilience estimation unit
- 300: Computer
- 301: CPU

## Claims

1. An information processing apparatus comprising:
an arousal state determination unit configured to determine which state among hyperarousal, optimal arousal, and hypoarousal an arousal state of a user is in.

2. The information processing apparatus according to claim 1, wherein
the arousal state determination unit determines the arousal state by calculating an arousal state level indicating the arousal state.

3. The information processing apparatus according to claim 2, wherein
the arousal state determination unit calculates the arousal state level on a basis of an activity degree of autonomic nerve of the user.

4. The information processing apparatus according to claim 3, wherein
the autonomic nerve includes a sympathetic nerve, a ventral vagus nerve, and a dorsal vagus nerve.

5. The information processing apparatus according to claim 4, wherein
the activity degree is a value obtained by scoring at least any of physiological index data, subjective index data, and behavioral index data of the user.

6. The information processing apparatus according to claim 5, wherein
the physiological index data includes at least any of a sensing result of each of utterance voice, a posture, a line-of-sight, expression, a brain wave, a saliva component, a hormone, and a vital sign of the user.

7. The information processing apparatus according to claim 5, wherein
the subjective index data includes at least any of a personality analysis result and a psychological test result for the user.

8. The information processing apparatus according to claim 5, wherein
the behavioral index data includes at least any of an analysis result of each of a motion, expression, a line-of-sight, and an utterance content of the user.

9. The information processing apparatus according to claim 1, further comprising:
a feedback information generation unit configured to generate feedback information for the user on a basis of the determined arousal state.

10. The information processing apparatus according to claim 9, wherein
the arousal state determination unit determines the arousal state in time series, and
the feedback information generation unit generates the feedback information in accordance with a fluctuation of the arousal state in a predetermined period.

11. The information processing apparatus according to claim 10, wherein
the feedback information generation unit generates the feedback information in accordance with a staying status of the arousal state in the hyperarousal or the hypoarousal.

12. The information processing apparatus according to claim 10, wherein
the feedback information generation unit generates the feedback information in accordance with a transition frequency of the arousal state between the hyperarousal and the hypoarousal.

13. The information processing apparatus according to claim 10, wherein
the feedback information generation unit generates the feedback information for guiding the arousal state of the user to the optimal arousal.

14. The information processing apparatus according to claim 9, further comprising:
a resilience estimation unit configured to estimate strength of resilience of the user on a basis of the arousal state, wherein
the feedback information generation unit generates the feedback information on a basis of the estimated strength of resilience.

15. The information processing apparatus according to claim 14, wherein
the resilience estimation unit estimates the strength of resilience on a basis of an accumulation result of the arousal state in a predetermined period.

16. The information processing apparatus according to claim 15, wherein
the resilience estimation unit estimates the strength of resilience from a threshold value of classification of the arousal state as the accumulation result.

17. The information processing apparatus according to claim 15, wherein
the resilience estimation unit estimates the strength of resilience from a distribution status of the arousal state as the accumulation result.

18. The information processing apparatus according to claim 14, wherein
the feedback information generation unit generates the feedback information for improving the strength of resilience.

19. An information processing method comprising
by an information processing apparatus:
determining which state among hyperarousal, optimal arousal, and hypoarousal an arousal state of a user is in; and
generating feedback information for the user on a basis of the determined arousal state.

20. A program for causing a computer to execute processing comprising:
determining which state among hyperarousal, optimal arousal, and hypoarousal an arousal state of a user is in; and
generating feedback information for the user on a basis of the determined arousal state.
